Europäisches Patentamt

(19) European Patent Office

Office européen des brevets

(11) **EP 0 918 296 A1**

(12) **EUROPEAN PATENT APPLICATION**

(43) Date of publication:
26.05.1999 Bulletin 1999/21

(51) Int. Cl.$^6$: **G06F 17/50**, G06F 17/30

(21) Application number: 97402620.5

(22) Date of filing: 04.11.1997

(84) Designated Contracting States:
AT BE CH DE DK ES FI FR GB GR IE IT LI LU MC
NL PT SE
Designated Extension States:
AL LT LV MK RO SI

(71) Applicant: Cerep
75007 Paris (FR)

(72) Inventor: Horvath, Dragos
59000 Lille (FR)

(74) Representative:
Becker, Philippe et al
Ernest Gutmann-Yves Plasseraud S.A.,
3, rue Chauveau-Lagarde
75008 Paris (FR)

Remarks:
A request for renumbering the claims 13 -16 to 12 -
15 has been filed pursuant to Rule 88 EPC. A
decision on the request will be taken during the
proceedings before the Examining Division
(Guidelines for Examination in the EPO, A-V, 3.).

(54) **Method of virtual retrieval of analogs of lead compounds by constituting potential libraries**

(57) The method to rapidly retrieve potentially active analogs of lead compounds according to the invention generates and screens from a large database of 3D multiconformational fingerprints of chemically feasible combinatorial products mainly by modeling the radicals by linking them temporarily to a bulky spacekeeper group, registering such 3D models of the radicals in a combinatorial ghost database, for any molecular structure that is accessible within the ghost database, detecting any atom that displays physical property features of the pharmacophoric type; for the pharmacophores (BPs) detected in each molecular structure, calculating all the distances between the involved atoms in every conformation of this molecule and creating a BP distance distribution density; generating a conformational fingerprint vector that contains all the distance distribution densities of the pharmacophores (BPs); defining a scoring function for each molecular fingerprint accounting for the relative importance of the pharmacophoric features; and comparing these fingerprints to each fingerprint of the potential library according to the above scoring function as maximized for the lead, and retrieving the molecules of the potential library for which the scoring function gives score values less than a specified threshold.

Step 10 — LIST OF PAIRS OF RADICALS

Step 11 — GHOST DATABASE
LOOPING OVER PAIRS
OF RADICALS (BP)

Step 12 — CONFORMER BONDING AND ROTATING

Step 13 — DISTANCE DISTRIBUTION DENSITY

Step 14 — CONFORMATIONAL FINGERPRINT OF FP AND MOLECULAR FINGERPRINT

III COMBINATORIAL "GHOST" DATABASE

Step 15 — LEAD FINGERPRINT VECTORS

Step 16 — SCORING FUNCTION SIMILARITY SCORE

Step 17 — SPECIFIED THRESHOLD SORTING FUNCTION

IV COMPARISON ALGORITHM

FIG_2

EP 0 918 296 A1

## Description

The invention relates to the combinatorial chemistry, and methods to screen and retrieve molecules from libraries that are structurally related with a lead compound to find synthetic analogs, e.g. analogs with similar biological or physical properties.

Virtual screenings, which evaluate similarity scores between each compound of a database and the reference or lead compound and retrieve such molecules, are already known. In particular, prediction of structures of active analogs, starting from a learning set of compounds of known biological activities is a research field in full development, where many different approaches have been reported and tested, such as the simulation of the "docking" of a ligand in a receptor site (Ajay & Murcko, J.Med.Chem., 388, pp. 681, 1995) or the free energy perturbation approaches (Kollmann, Chemical Revue 1993, pp. 2395, 1995), or the screening 3D approaches (Tripos Technical Notes, Vol. 1, Nr.2 - Molecular Diversity Manager, October 1995, as well as the program Cerius2 Drug Discovery Workbench from "MSI" Inc, Molecular Simulations Incorporated).

However, the instant problem to be solved is the retrieval of similar, potentially active analogs for molecules of known biological activity out of the large collection of hundreds of millions of combinatorial products, synthetised on hand of building blocks of a reference library and of available chemical know-how, in order to select a biased sublibrary having a maximum content in active compounds and which can be synthetised and tested with respect to the presumed biological activity.

Prior art approaches are either too time-consuming or non-realistic in order to provide both fast and accurate retrieval of active analogs that are chemically feasible.

Recently published 2D approaches can be used to describe large libraries of molecules in term of connectivity descriptors, such as the issue by Higgs, Bemis, Watson & Wikel in J.Chem.Inf.Comp. Sci. Vol 37 n°5, pp. 861, 1997. However they donot account for geometrial and conformational aspects. Besides, approaches which analyze the molecular connectivity of large sets of candidates, are often flowed by their lack of realism in the description of the molecules.

More realistic approaches, like Tripos's approach, called 《COMFA》(Comparative Molecular Fields Analysis), require an unambiguous superposition of the compared molecules, i.e. only fairly similar compounds (compounds having a common "template" or skeleton) can be meaningfully compared to each other. Furthermore, a great uncertainty remains on whether the calculated superposition mode of the compared molecules is physically relevant with respect to the binding mode to a receptor.

Furthermore, most of the prior art approaches perform retrieval of active analogs out of more or less random collections of products which oftenly leads to situations where the retrieved molecules are not chemically synthetisable, unstable or generally inadequate for use as drugs.

The present invention aims to solve these problems and propose a new approach based on an optimized trade-off between the degree of realism of the description of the molecules and the rapidity of retrieving them, by generating potential libraries that encode 3D multiconformational information under the form of pharmacophoric fingerprints of combinatorial products, and screening of these libraries by using scoring functions with a number of parameters specifically chosen.

More precisely, the object of the present invention is a method to rapidly retrieve potentially active analogs of lead compounds, wherein 3D fingerprints are generated and screened from a large database of 3D multiconformational fingerprints of chemically feasible combinatorial products as follows:

- selecting systematically by a chemical filter algorithm, according to elementary chemical rules, couples of radicals each including a reactive center, as valid reaction partners, from Building Blocks (BBs) constituents of a reference library, the reactive centers being detected by analysing the molecular connectivity and the leaving groups being deleted;

- modeling the radicals by linking them temporarily to a bulky spacekeeper group and submitting the resulting complex to conformational sampling run yielding a collection of conformers, then removing the spacekeeper in order to ensure that the reactive centers of the radicals are sterically accessible and its free valency points towards a region of free space previously occupied by the spacekeeper;

- registering such 3D models of the radicals in a combinatorial ghost database, after verifying that these models comply with the coupling ability according to sterical hindrance and conformational diversity criteria of the ghost database, this ghost database emulating a database of combinatorial products in instantly generating the structure of any such product by linking together the registered structures of the constituting radicals;

- for any molecular structure that is accessible within the ghost database, detecting any atom that displays physical property features of the pharmacophoric type, involved in determining the intensity of intermolecular interactions, at least the hydrophobic, aromatic, hydrogen bond donor, hydrogen bond acceptor, anionic or cationic characters, on the basis of elementary rules accounting for the chemical nature of such an atom and the molecular environment in which it is placed, and listing all the possible bipolar pharmacophores pairs (BP) in which each atom displays a given pharmacophoric feature;

- for the pharmacophores (BPs) detected in each molecular structure, calculating all the distances between the involved atoms in every conformation of this molecule and creating a BP distance distribution density, by fuzzy monitoring of the number of BPs for which the associated distances fall within given ranges;
- generating a conformational fingerprint vector that contains all the distance distribution densities of the pharmacophores (BPs) associated to a current conformation, obtaining an average molecular fingerprint from the conformational fingerprints of the considered conformers to constitute and supply a potential library,
- defining a scoring function for each molecular fingerprint accounting for the relative importance of the pharmacophoric features owings to weighting factors that are calibrated in order to maximize a discriminative power with respect to different binding affinities; and
- generating the fingerprints of the lead for which analogs are to be retrieved, comparing these fingerprints to each fingerprint of the potential library according to the above scoring function as maximized for the lead, and retrieving the molecules of the potential library for which the scoring function gives score values less than a specified threshold.

In particular embodiments, the weighting factors of the scoring function are calibrated in order to maximize the discriminative power between families of ligands of different receptors in a so-called 《General Diversity paradigm》, or to maximize the discriminative power between the compounds that bind to a given receptor, in contrast to those that have no binding affinity with respect to it, in a so-called 《Receptor-Oriented Diversity paradigm》.

In a prefered embodiment, the method of the present invention includes, at the stage of generating the potential library, preliminary checking steps in order to discard the pairs of building blocks that can not be used as partners in any of the available synthesis protocols, either due to the absence of appropriate reactive groups or due to the presence of potentially interfering groups that may trigger unwanted side reactions, in order to prevent that the generated potential library does not contain compounds which could be formally represented as the coupling products of two BBs, but which for chemical reasons cannot be obtained in that way.

Therefore, all the molecules composing the "potential library" are actually synthetisable and represent pharmacologically acceptable species (without "exotic", very reactive or unstable groups), with the direct consequence that any of analogs retrieved by the virtual screening of the invention can be synthetised with little effort.

The method according to the present invention allows to design biased libraries that include the herein retrieved analogs, synthetizing these biased libraries and evaluating the activities of their products. Furthermore, the herein generated structure-activity data can be used in order to improve the parametrization of the scoring function or to initiate new predictive approaches such as neural networks, able to estimate the required activity of the molecule on the basis if its fingerprint.

There are several advantages of present approach, in contrast to other drug design strategies and information management schemes in combinatorial chemistry.

The method according to the present invention can be integrated to a discovery paradigm that does no longer need a primary, "blind" screening of a compound library, if at least one ligand structure is known for the studied receptor.

Such paradigm includes the steps of generating and updating the potential library of fingerprints of combinatorial compounds, retrieval of potentially active analogs of known ligands out of this potential library, on the basis of the 《General Diversity》 paradigm, and design of a biased sublibrary, synthesis of the biased library and identification of active compounds, training and adjusting the parameters in order to define the 《Receptor-Oriented Diversity》 scoring function, or to obtain a predictive neural network, retrieval and synthesis of other potentially active according to the previously calibrated scoring function or neural network.

The potential library is directly linked to the building block stock databases, and updated in function of the changes in available building blocks and validated chemistries; this is a net advantage over the concept of virtual libraries which contain more or less random selections of compounds that may or may not be chemically feasible and/or pharmacologically interesting,

The build-up of the fingerprints stored in the potential library takes profit of the extremely fast access to the multiconformational models of the combinatorial products from the combinatorial ghost database, which precludes the need of an explicit generation of three-dimensional for the up to 100 million product molecules; using one of the fastest conformational sampling programs, such the software "Catalyst" of MSI (Molecular Simulations Incorporation) that is claimed to process up to 10.000 compounds per 24 CPU hours, largely more than 1000 days would be required to complete such a task.

The combinatorial ghost database offers immediate access to the multiconformational models of any combinatorial product and instantly generates them by linking together the registered conformers of the radicals that constitute this product, and performing a 2 or 3-step torsional angle driving around the newly formed bond. The obtained conformers of the product are free of interatomic clashes, due to the precautions taken when modeling the radicals prior to their registration in the ghost database.

An explicit check of the quality of the geometries obtained by the coupling is nevertheless performed, in spite of those precautions. This operation is hardly more time-consuming than the input of molecular data files of the product : access to the structures of (MxN) combinatorial products of a combined type that can be obtained out of M BBs of a first type and N BBs of a second type is gained at the cost of a conformational sampling effort required to obtain the 3D-models of the (M+N) BBs.

The generated fingerprints represent distance distribution densities between pairs of atoms matching a pair of given pharmacophoric features. The rule-based identification of the atoms displaying given pharmacophoric features being very fast, the generation of the fingerprints of 100 million compounds would take some tens days depending on the processor operating system and its peripheral, for instance an estimated 20 to 30 days on a Unix workstation. They can be used to describe individual conformations, molecules, as well as collections of molecules within a unified formalism. Histograms corresponding to these fingreprints can be straightforwardly plotted and interpreted.

Other advantages and features of the present invention will be disclosed in the hereafter detailed description of non imitative embodiment in reference with the annexed figures which respectively show:

- Figure 1, a schematic flowchart of a first steps series of an exemplified method according to the invention and relative to a chemical algorithmic filter to select couples of radicals;
- Figure 2, a schematic flowchart of the following step series of such a method relative to a geometrical algorithmic filter to provide and register 3-D models of the radicals in a ghost data base;

The construction of potential libraries according to the invention relies on the collection of building blocks, refered as BB, currently available from a library and a set of synthesis protocols, both of which are regularly updated. Each such update automatically triggers the update of the potential libraries. A database containig the molecular 2D-sketches of BB, furnishes a description of the molecular connectivity of the BB.

Each synthesis protocol preferably requires, in the initial BB molecules, the presence of appropriate functional groups and the absence of potentially interferring reactive groups which may lead to side reactions. A preliminary algorithm selects its required molecules in respect of their chemical compatibility defined in each "reactivity profile" depending on the considered synthesis protocol, the chemical properties of the ligands of the receptor or the binding ability to the receptor. Such an algorithm is in the scope of the art of the skilled person.

Furthermore, each synthesis protocol may involve functional "transformers" to be appended to a first BB, prior to its coupling to a second BB. Thus, the chemical reactions hereafter considered are:

- either direct coupling processes between building blocks, such as for instance, with R1-COOH as a first BB, and $H_2N$-R2 as a second BB:

$$R1\text{-}COOH + H_2N\text{-}R2 \rightarrow R1\text{-}CO\text{-}HN\text{-}R2 + HOH$$

- or coupling with a functional transformation of the first BB prior to the coupling to the second BB, such as in:

$R1\text{-}NH_2 + Im\text{-}CO\text{-}Im \rightarrow R1\text{-}NH\text{-}CO\text{-}Im + HIm$ (Im=imidazole) $R1\text{-}NH\text{-}CO\text{-}Im + H_2N\text{-}R2 \rightarrow R1\text{-}NH\text{-}CO\text{-}NH\text{-}R2 + HIm$

Transformers (the carbonyl group -C(=O)- in the above example) replace the original reactivity by a new one, opening the possibility to use the modified BBs in synthesis that are not feasible with the original ones.

A first algorithmic filter, referred to as the chemical filter, is implemented in order to check whether each BB qualifies for a given reaction, according to reactivity specifications listed in the corresponding synthesis protocol.

The chemical filter is used to select two subsets of BBs of type A and respectively B, which are considered to be valid reaction partners, to yield products of the type A-T-B, with T being the transformer, if any, required by the considered chemical assembly strategy. It involves preliminary steps to 〈〈clean〉〉 the BBs in removing accompanying counterions and cutting away the leaving groups to form radicals in which the reactive center identified as such.

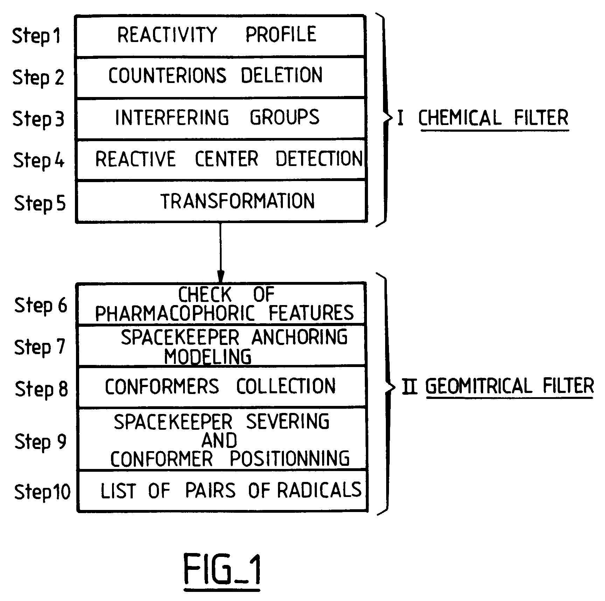

An example of a chemical filter I is illustrated in figure 1. It comprises steps 1 to 3 as preliminary steps and steps 4 and 5 as conformational steps, as follows.

Step 1 : scanning the so-called "reactivity profile" of the current synthesis, i.e. required groups specifying, in consideration of their reactivity in view of their bonding ability with partners to make the synthesis possible, of interferring groups and transformers

- the type of the reactive center or the degree of substitution of nucleophilic centers (e.g. primary and secondary monoaromatic amines) out of lists of several choices,

- thresholds values of number of rotatable bonds (flexibility threshold) and of the molecular mass, and then selecting the BB files.

Step 2 : checking if the selected BB contains a single molecule and deleting the counterions defined as maximal size of connex subgraphs (such as $R\text{-}NH_3^+,/Cl^-$) if any;

Step 3 : checking the presence of any interferring groups in the selected BB that should trigger secondary processes, and discarding such current BB;

Step 4 : checking the presence of the required functional groups in the BB, and figuring out which atom, called hereafter the reactive center, is involved in forming a bond with the second BB. A part of the BB, called the "leaving group", is eliminated during the reaction to prepare the reactive center to be linked, the step detecting and deleting the corresponding fragment.

For example: in a carboxylic acid in an amidificaiton process, the leaving group -OH is deleted and the reactive center is set at the carboxyl carbon:

$$R\text{-}C(=O)\text{-}OH \rightarrow R\text{-}C^*(=O)\text{-},$$

*labels the reactive center.

If the BB contains several potentially reactive groups, then all the possible reactive centers are enumerated and a selection of the correct one is conducted with auxiliary software or routine rules implemented by the skilled person, or the compound is discarded; if no reactive group is found, the compound is also discarded.

Step 5 : if a functional transformation is specified due to the fact that another reactive center is considered to be more tunable to the synthesis, the transformer fragment is attached to the previously detected reactive center and a new reactive center is located at the atom of the transformer fragment that will form a bond with the BB, as, for example, with a reaction of the type:

$$R\text{-}HN^*\text{-}+\text{-}C(=O)\text{-} \rightarrow R\text{-}HN\text{-}C^*(=O)\text{-}$$

Thus, the chemical filter transforms the raw structures of the BBs into corresponding fragments as they appear in a final product, in detecting the reactive centers, deleting the leaving groups and coupling to a transformer moiety when required. These fragments are referred to as "radicals" which are liable to be reaction partners referred to as S1 and S2, one of the valencies of the reactive center is labeled as the free valency, to be used for coupling with the partner radicals in order to obtain the final products.

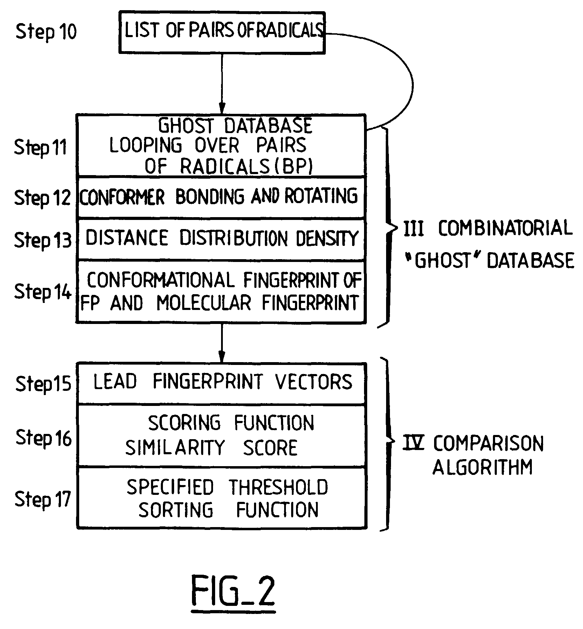

In the following rule-based algorithm II, a geometrical filter as illustrated in figure 2, the construction of 3D-geometries from conformational sampling of selected radicals, are carried out such as to ensure that, in resulting geometries, the reactive centers of the radicals are sterically accessible, e.g. the free valency points towards a region of free space, in order to ensure that any radical can be concatenated with partner radicals without any clashes between the linked moieties which form the final product.

In order to achieve this, a bulky "spacekeeper" group linked to the selected radicals is used in connection with the conformational sampling. In the present embodiment (figure 2), the following steps are carried out.

Step 6: hydrogens being added to the heavy atom skeleton of the sketches of each radical, 6 pharmacophoric features: aromaticity, hydrophobicity, hydrogen bond donor or acceptor property, positive and negative charge of every atom, are checked and listed, considering the specific chemical groups involved and more particularly that

- aliphatic amino groups are under cationic form, while aromatic amines are taken as neutral;
- a special flag is used to signal the presence of imidazole rings, which may appear under physiological conditions, at neutral pH, under both protonated or unprotonated form;
- carboxylate, sulphonate, phosphate groups and tetrazoles are considered to be anions.

Step 7: the 2D-sketch of the radical is anchored with its free valency to the following bulky spacekeeper group, the tris(triiodosilyl)methyl-entity:

$$R\text{-}HN\text{-}C^*(=O)\text{-}+\text{-}C(SiI_3)_3 \rightarrow R\text{-}HN\text{-}C(=O)\text{-}C(SiI_3)_3$$

Step 8: the resulting 2D-sketch of this compound is submitted to a conformational sampling run, performed for instance by the "Catalyst" MSI software, yielding a collection of possible conformers of this compound.

Step 9: for each of the conformers obtained at step 8, the spacekeeper moiety is now severed and deleted, restoring the free valency of the radical which now points towards the empty region previously occupied by the spacekeeper.

Advantageously, steric hindrance criteria are then performed in order to check whether the spacekeeper strategy has managed to insure an appropriate accessibility for the reactive center for every conformation; the criteria are for instance:

- a comparison of the interatomic distances in the different conformations in order to make sure that the retained conformers are not redundant;
- an energy criterion discarding higher-energy conformers found to have an almost identical geometry with respect to lower-energy conformations.

At step 9, for each retained conformer, a coordinate transformation in a 3-Dimensional OXYZ reference system is performed, in order to place the reactive center in the origin of the reference system and to align the free valency along the Z-axis. The coordinates of the conformers are stored.

Step 10: a list of all the potential compounds that are obtained by first coupling each radical of first sets S1 with every one of its partners S2 is then generated.

This list serves as input for a "combinatorial explosion" ghost database of fingerprints and is submitted as a query to such a combinatorial ghost database which rapidly generates the structures of the herein enumerated dimers by linking every conformer of the BBs of a first type to every conformer of the BBs of a second type, performing a 2 or 3-step torsional angle driving around the linkage bond, evaluating the distances within each of the so generated conformers.

The minimal distance between any two heavy atoms belonging to the initial radicals and separated by more than three bonds in the final molecule is evaluated and is checked if it is above a predetermined threshold. If tu=his is not the case, that particular conformer of the dimer is discarded.

A distance distribution density of pairs of pharmacophoric features and interatomic distances is constructed. The matching to a given pair of pharmacophoric features and the reaching to interatomic distances in a given range value constitute conformational fingerprint vectors, each fingerprint element of them being the number of combinations that can be obtained with the pharmacophoric features.

A molecular fingerprint vector is then obtained by summation of the conformational fingerprints, followed by norming this sum with respect to the numbers of considered conformers of the product to provide an average molecular fingerprint.

In the present embodiment, a combinatorial amplifier algorithm III constituting a 《ghost》database (figure 2) recites this combinatorial explosion by carrying out the following steps:

- in step 11, looping over all pairs listed at step 10, current pairs of radicals, the first from S1, the second from S2, are stored together with all their available conformers C, as obtained at step 9 and the lists of pharmacophoric features of the composing atoms formerly used. The pharmacophoric features of the atoms that are linked by new bonds are reevaluated, since their chemical type have changed due to this chemical transformation;
- in step 12, each conformer C1 of the first radical S1 is linked with each conformer C2 of the second radical S2, in mirroring the coordinates of the latter with respect to the XOY plane, and translating them along the Z axis in order to restore the correct length of the newly formed bond between C1 and C2; a 2 or 3-step rotation around the new axis is performed.

    Therefore, the generated number of conformers of the coupling product equal the number of conformers of the first times that of the second radical, times the number of rotations around the newly formed bond;
- in step 13, in order to construct the distance distribution density of pairs of pharmacophoric features, a complete set of interatomic distances is evaluated for the current conformation of each dimer; all the distances between pairs of atoms matching a given pair of pharmacophoric features and taking a value between n and n+1 Angstroms, n being an integral number, are counted;
- in step 14, a generated conformational fingerprint vector describing the current geometry is represented, in the present embodiment, with a (6x7)/2x12=252-element vector, where (6x7)/2=21 is the number of combinations that can be obtained with the 6 pharmacophoric features f1 to f6 introduced supra (aromatic-aromatic, aromatic-hydrophobic, ..., anion-anion).

Each fingerprint element FP (fa, fb, i), fa and fb being one of the group f1 to f6, is equal to the number of atoms pairs matching a given pair of pharmacophoric features (fa,fb) and which are separated by a distance between i+3 and i+4 Angstroms, i being an integral number. For example, the element of the fingerprint FP(cation,aromatic, 1) counts the number of atoms pairs in which one is a cation, the other is an aromatic atom and the distance between them falls within the range 4 to 5 Angstroms.

A molecular fingerprint vector is obtained by summation of the conformational fingerprints, followed by norming this sum with respect to the numbers of considered conformers of the product. This fingerprint vector is stored in association with the identification codes of the two radicals which compose that product. The collection of all the fingerprints of all the possible coupling products between all BBs qualifies the initial library synthesis processes and defines a potential library.

A comparison algorithmic filter IV is then performed to compare the fingerprints of the reference compounds with each fingerprint of the potential library by encoding of leads and known ligand structures, by comparing the distance distributions corresponding to each pair of features with partial scores similarity score per feature pair and a similarity score.

Weighting factors are introduced to represent the relative importance of the different pharmacophoric features, and are the tunable parameters of the method. Indeed, the different physical, chemical or biological properties are more sensitive to the presence of specific bipolar pharmacophores than others : the similarity of two compounds with respect to given feature pairs is more important, while the that the fact that the two compounds differ with respect to other feature pairs. To reflect the relative importance of the feature pairs, the weighting factors wieights the the partial scores in the calculation of the overall similarity score.

In the present embodiment, the comparison algorithm IV (figure 2) consists of :

- encoding, in step 15, of leads or known ligand structures, under the form of fingerprints, following the same procedure as herebefore described, except for the fact that the used conformers are those directly generated by the run "Catalyst" soft of MSI algorithm, to which the sketches of these reference compounds are submitted as such;
- introducing a scoring function, in step 16, which successively compares the distance distributions corresponding to each pair of features (fa, fb).

First, the 21 partial scores expressed as pnorm1(fa,fb), pnorm2(fa,fb) and pcross(fa,fb), are calculated in the form of convolution products for every pair of features, as follows

$$\text{pnorm1(fa,fb)} = \sum_{i,j=1\ldots.12} \text{FP\_mol1(fa,fb,i)*FP\_mol1(fa,fb,j)}* e^{-(i-j)*(i-j)}$$

$$\text{pnorm2(fa,fb)} = \sum_{i,j=1\ldots.12} \text{FP\_mol2(fa,fb,i)*FP\_mol2(fa,fb,j)}*e^{-(i-j)*(i-j)}$$

$$\text{pcross(fa,fb)} = \sum_{i,j=1\ldots.12} \text{FP\_mol1(fa,fb,i)*FP\_mol2(fa,fb,j)}*e^{-(i-j)*(i-j)}$$

where FP_mol1 and FP_mol2 are the fingerprints of the first compound, a reference one, and, respectively, the second compound, a tested one, i and j are variables looping over all the considered distance bins, as described at step X, and $\alpha$ an exponential damping factor.

If pnorm1(fa,fb) and pnorm2(fa,fb) are simultaneously zero, it means that the corresponding pairs of features do not occur in any one of the molecules; therefore, such combinations are ignored when evaluating the global similarity score between mol1 and mol2. Otherwise, the partial similarity score per feature pair, psim(fa,fb) is defined by

$$\text{psim(fa,fb)} = 2\text{pcross(fa,fb)}/[\text{pnorm1(fa,fb)}+\text{pnorm2(fa,fb)}]$$

And the similarity score by a sim-score which involves a weighting factor:

$$\text{sim-score} = 1-[\,\Sigma\,\text{W(fa)W(fb)psim(fa,fb)}]/[\,\Sigma\,\text{W(fa)W(fb)}]$$

where W(f) is the weighting factor for the feature f.

In the sim-score expression, both sums are taken over the feature (fa,fb) for each at least one of pnorm1(fa,fb) and pnorm2(fa,fb) are not zero, e.g. the pairs that appear in at least one of the two molecules.

The weighting factors W(f) are the tunable parameters of the method together with the exponential damping factor that controls the value of pnorm1, pnorm2, pcross. The values of such tunable parameters are obtained by different calibration approaches, aimed to optimize the overall performance of the model.

The number of fittable factors can be reduced to restrain the number of degrees of freedom of the 21-dimensional space, n the case of six pharmacophoric features. In this case, the simplification recites:

$$W(fa,fb) = W(fa) . W(fb)$$

Then, the weighting factors assocated to the bipolar pharmacophores are written as a product of thevweighting factors : the search for the 21 optimal factors values is reduced to asearch of only 6 values.

- listing, in step 17, all the molecules mol2 of the potential library for which their similarity score, sim-score, with respect to the reference compound mol1 is less than a specified threshold with a sorting function in respect of the similarity score value. Synthesis of the retrieved structures or the best of them are performed from the potential library, and subject to biological testing. Alternatively or cumulatively, a list of all the building blocks represented in the retrieved products is established and a generation focussed combinatorial library is based on such BBs.

Two approaches are possible according to the specific search to be carried out, the General Diversity paradigm and the Receptor-Oriented paradigm..

The General Diversity paradigm consists in choosing the weighting factors in order to obtain a similarity scoring function which successively discriminates between classes of ligands of different receptors. Given an arbitrary collection of clusters of ligands associated to different receptors, eah cluster consisting of families of ligands that exclusively bind to the associated receptor, the《most diverse》subset of ligands retrieves one ligand per receptor if the scoring function on which this most diverse subset selection has been realized has an ideal disriminative power. Because a less disriminating function would selet several ligands of the same receptor, while completely ignoring other ligand families, the weighting are optimized in order to improve the discriminative power of the distances between two molecules by using as an objective function the number of receptors for which at least one ligand has been piked out in the most diverse selection. Then the obtained weighting factors values characterize, if the number of receptors is sufficiently large, the aerage propensities of the bipolar pharmacophores to contribute to the anchoring of a ligand in a receptor site.

As to illustrate this paradigm, six reference ligands of the DAT receptors (dopamine carrier / IDM) have been used to carry out a first modelisation. Compounds of different chemistries, chemistry of functionnel rearrangements, reducive amination, amides, urea, carbamates and esters, have been sorted according to the filters and the general diversity paradigm according to the present method. 42 compounds have been screened and synthesized providing from a 21 aldehydes vector and a 2 acids vector. Among them, 14 compounds have an inhibition bond (which measure the stability of the bond) beyond 50%, with 4 beyond 70% and one above 90% (93%).

The Receptor-Oriented Diersity paradigm consisting in calibrating the weighting factors on the basis of primary screening results of a library against a given receptor, such as to minimize the average distance between any two active compounds and to maximize the dissimilarity scores between each active and any inactive compound. This calibration mode allows to define which pharmacophores are essential for the binding to a given receptor.

According to an example, analogs to the serotomine activity on the $5\text{-HT}_4$ receptor (ability of compounds to inhibit $5\text{-HT}_4$ induced contractions of guinea-pig ileum preparations) have been modelised according the receptor-oriented divresity score function of the invention. 60 cabamates, 35 esters , 4 ureas, and one amide have been retrieved. The activity (inhibition above 50%) of 14 compounds have been cofirmed.

The invention is not limited to the examples as described and illustrated. In particular, different reference libraries corresponding to different chemistries can supply the reference library of the Bbs. In other respects, the present invention can be applied to diffent contexes, for instance to search analogs to a given product having specified chemical properties (a detergent, ...).

**Claims**

1. Method to rapidly retrieve potentially active analogs of lead compounds, wherein 3D fingerprints are generated and screened from a large database of 3D multiconformational fingerprints of chemically feasible combinatorial products as follows:

- selecting systematically by a chemical filter algorithm, according to elementary chemical rules, couples of radicals each including a reactive center, as valid reaction partners, from Building Blocks (BBs) constituents of a reference library, the reactive centers being detected by analysing the molecular connectivity and the leaving groups being deleted;

- modeling the radicals by linking them temporarily to a bulky spacekeeper group and submitting the resulting complex to conformational sampling run yielding a collection of conformers, then removing the spacekeeper in order to ensure that the reactive centers of the radicals are sterically accessible and its free valency points towards a region of free space previously occupied by the spacekeeper;
- registering such 3D models of the radicals in a combinatorial ghost database, after verifying that these models comply with the coupling ability according to sterical hindrance and conformational diversity criteria of the ghost database, this ghost database emulating a database of combinatorial products in instantly generating the structure of any such product by linking together the registered structures of the constituting radicals;
- for any molecular structure that is accessible within the ghost database, detecting any atom that displays physical property features of the pharmacophoric type, involved in determining the intensity of intermolecular interactions, at least the hydrophobic, aromatic, hydrogen bond donor, hydrogen bond acceptor, anionic or cationic characters, on the basis of elementary rules accounting for the chemical nature of such an atom and the molecular environment in which it is placed, and listing all the possible bipolar pharmacophores pairs (BP) in which each atom displays a given pharmacophoric feature;
- for the pharmacophores (BPs) detected in each molecular structure, calculating all the distances between the involved atoms in every conformation of this molecule and creating a BP distance distribution density, by fuzzy monitoring of the number of BPs for which the associated distances fall within given ranges;
- generating a conformational fingerprint vector that contains all the distance distribution densities of the pharmacophores (BPs) associated to a current conformation, obtaining an average molecular fingerprint from the conformational fingerprints of the considered conformers to constitute and supply a potential library,
- defining a scoring function for each molecular fingerprint accounting for the relative importance of the pharmacophoric features owings to weighting factors that are calibrated in order to maximize a discriminative power with respect to different binding affinities; and
- generating the fingerprints of the lead for which analogs are to be retrieved, comparing these fingerprints to each fingerprint of the potential library according to the above scoring function as maximized for the lead, and retrieving the molecules of the potential library for which the scoring function gives score values less than a specified threshold.

2. Method according to claim 1, wherein the weighting factors of the scoring function are calibrated in order to maximize the discriminative power between families of ligands of different receptors in a 《General Diversity paradigm》 by using as an objective function the number of receptors for which at least one ligand has been piked out in the most diverse selection.

3. Method according to claim 1, wherein the weighting factors of the scoring function are calibrated in order to maximize the discriminative power between the compounds that bind to a given receptor, in contrast to those that have no binding affinity with respect to it, in a 《Receptor-Oriented Diversity paradigm》 by primary screening results of a library against a given receptor, such as to minimize the average distance between any two active compounds and to maximize the dissimilarity scores between each active and any inactive compound.

4. Method according to anyone of the preceeding claims, characterised in that preliminary checking steps in order to discard current building blocks which could include potentially interfering reactive groups and/or to add functional groups to a building block prior coupling are included.

5. Method according to anyone of the preceeding claims, characterised in that the yielding of conformers 3D is completed with an encoding structural information step over a geometrical filter, by extracting from a conformational fast sampling analysis of the molecular structures of 2D-sketches of the selected radicals in connection with pharmacophoric features and with respect to each specific chemical group involved in each radical to detect and discard remaining conformations of important steric hindrance around the reactive center and conformations similar to other sampled ones.

6. Method according to anyone of the preceeding claims, characterised in that the building blocks currently available from a reference library and a set of synthesis protocols are regularly updated, each such update automatically triggering the update of the potential libraries.

7. Method according to claim 1, characterised in that it is integrated to a discovery paradigm defining a feedback loop starting from a primary, "blind" screening of a compound library, and including the steps of identification of active compounds, training and adjusting parameters of a predictive model to recognize the specific features of active compounds, retrieval of potentially active analogs for these compounds out of a reference library according to the instant method, synthesis of these analogs and screening to refine the parameters.

8. Method according to anyone of the preceeding claims, characterised in that it comprises essentially the following steps:

- scanning a reactivity profile of the current synthesis consisting of required groups and interferring groups (step 1);
- checking if the selected BB contains a single molecule and deleting the counterions (step 2);
- checking the presence of any interferring groups in the selected BB and discarding such current BB (step 3);
- checking the presence of required functional groups in the BB and figuring out the reactive center involved in the bond with a second BB to form reactive partner radicals (S1,S2), eliminating the leaving groups (step 4);
- adding hydrogens to the heavy atom skeleton of the sketches of each radical (S1,S2), at least six pharmacophoric features, aromaticity, hydrophobicity, hydrogen bond donor or acceptor property, positive and negative charge of every atom, being checked and listed (step 6);
- anchoring the 2D-sketch of the radical with its free valency to the following bulky spacekeeper group (step 7);
- submitting the resulting 2D-sketch of this compound to known conformational sampling run, yielding a collection of conformers (C) (step 8);
- severing and deleting, for each of the conformers obtained (step 8), the spacekeeper moiety, restoring the free valency of the radical which now points towards the empty region previously occupied by the spacekeeper (step 9);
- performing, for each retained conformer (C), a coordinate transformation in a reference system (OXYZ), in order to place the reactive center in the origin of the reference system and to align the free valency along the Z-axis (step 9);
- generating a list of all the compounds that are obtained by coupling each radical of first sets (S1) with every one of its partners (S2) (step 10);
- looping over all pairs listed (step 10) and storing current pairs of radicals (S1, S2) together with all their available conformers (C), as obtained (step 9) and the lists of pharmacophoric features of the composing atoms formerly used, the pharmacophoric features of the atoms linked by new bonds being reevaluated (step 11)
- linking each conformer (C1) of the first radical (S1) with each conformer (C2) of the second radical (S2) in minoring the coordinates of the latter with respect to the XOY plane, translating them along an axis (Z) in order to restore the correct length of the newly formed bond between the conformers (C1, C2); and rotating around the new axis (step 12);
- in order to construct the distance distribution density of pairs of pharmacophoric features, evaluating a complete set of interatomic distances for the current conformation of each dimer; all the distances between pairs of atoms which match a given pair of pharmacophoric features and taking a value between n and n+1 Angstroms being counted (step 13);
- representing a generated conformational fingerprint vector describing the current geometry with a number of elements with respect to the number of combinations that can be obtained with the pharmacophoric features, each fingerprint element (FP) being equal to the number of atoms pairs matching a given pair of pharmacophoric features (fa,fb) and which are separated by a distance between i+3 and i+4 Angstroms (step 14);
- summing the conformational fingerprints to obtain a molecular fingerprint vector, followed by norming this sum with respect to the numbers of considered conformers of the product, each fingerprint vector being stored in association with the identification codes of the two radicals which compose that product; the collection of all the fingerprints of all the possible coupling products between all BBs qualifying the initial synthesis processes and defining potential libraries. (step 14);
- encoding of leads or known ligand structures, under the form of fingerprints, following the same procedure directly generated by a run conformational algorithm, to which the sketches of these reference compounds are submitted as such (step 15);
- introducing a scoring function, which successively compares the distance distributions corresponding to each pair of features (fa, fb). (step 16) and
- listing all the molecules (mol2) of the potential library for which their similarity score (sim-score), with respect to the reference compound (mol1) is less than a specified threshold owings to a sorting function in respect of the similarity score value (step 17).

9. Method according to claim 4, characterised in that specific chemical groups involved are

- aliphatic amino groups are under cationic form, while aromatic amines are taken as neutral;
- a special flag is used to signal the presence of imidazole rings, which may appear under physiological conditions, at neutral pH, under both protonated or unprotonated form;
- carboxylate, sulphonate, phosphate groups and tetrazoles are considered to be anions.

10. Method according to claim 5, characterised in that steric hindrance criteria are then performed in order to check whether the spacekeeper strategy has managed to insure an appropriate accessibility for the reactive center for every conformation; the criteria are for instance:

- a comparison of the interatomic distances in the different conformations in order to make sure that the retained conformers are not redundant;
- an energy criterion discarding higher-energy conformers found to have an almost identical geometry with respect to lower-energy conformations.

**11.** Method according to claim 8, characterised in that each conformer (C1) of the first radical (S1) is linked with each conformer (C2) of the second radical (S2), in minoring the coordinates of the latter with respect to a plane (XOY) of the reference system, translating them along the axis (Z) perpendicular to that plane (XOY) in order to restore the correct length of the newly formed bond between the conformers ( C1, C2); and rotating them around the new axis.

**13.** Method according to anyone of the preceeding claims, characterised in that the reactivity profile includes transformers to be coupled to the reactive center of a first BB prior to its coupling to the second BB, each synthesis protocol involving functional transformers to be appended to the first BB.

**14.** Method according to anyone of the preceeding claims, characterised in that the bulky spacekeeper group is a tris(triiodosilyl)methyl-entity.

**15.** Method according to anyone of the preceeding claims, characterised in that, the number of pharmacophoric features being six, a generated conformational fingerprint vector describing the current geometry is represented,, with a 252-element vector,in respect of the number of combinations obtained with six features.

**16.** Method according to anyone of the preceeding claims, characterised in that synthesis of the retrieved structures are performed from the potential library, and subject to biological testing, and that a list of all the BBs represented in the retrieved products is established and a generation focussed combinatorial library is based on such BBs.

| | |
|---|---|
| Step 1 | REACTIVITY PROFILE |
| Step 2 | COUNTERIONS DELETION |
| Step 3 | INTERFERING GROUPS |
| Step 4 | REACTIVE CENTER DETECTION |
| Step 5 | TRANSFORMATION |

I CHEMICAL FILTER

| | |
|---|---|
| Step 6 | CHECK OF PHARMACOPHORIC FEATURES |
| Step 7 | SPACEKEEPER ANCHORING MODELING |
| Step 8 | CONFORMERS COLLECTION |
| Step 9 | SPACEKEEPER SEVERING AND CONFORMER POSITIONNING |
| Step 10 | LIST OF PAIRS OF RADICALS |

II GEOMITRICAL FILTER

## FIG_1

Step 10 | LIST OF PAIRS OF RADICALS

Step 11 | GHOST DATABASE
LOOPING OVER PAIRS
OF RADICALS (BP)

Step 12 | CONFORMER BONDING AND ROTATING

Step 13 | DISTANCE DISTRIBUTION DENSITY

Step 14 | CONFORMATIONAL FINGERPRINT OF
FP AND MOLECULAR FINGERPRINT

III COMBINATORIAL
"GHOST" DATABASE

Step 15 | LEAD FINGERPRINT VECTORS

Step 16 | SCORING FUNCTION
SIMILARITY SCORE

Step 17 | SPECIFIED THRESHOLD
SORTING FUNCTION

IV COMPARISON
ALGORITHM

## FIG_2

European Patent Office

**EUROPEAN SEARCH REPORT**

Application Number

EP 97 40 2620

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | PICKETT ET AL: "diversity profiling and design using 3d pharmacophores : pharmacore-derived queries (PDQ)" JOURNAL OF CHEMICAL INFORMATION AND COMPUTER SCIENCES., vol. 36, 1996, WASHINGTON US, pages 1214-1223, XP002062096 * the whole document * | 1 | G06F17/50 G06F17/30 |
| A | EP 0 790 567 A (ITAI AKIKO) * the whole document * | 1 | |
| A | MARTIN Y C: "3D DATABASE SEARCHING IN DRUG DESIGN" JOURNAL OF MEDICINAL CHEMISTRY, vol. 35, no. 12, 12 June 1992, pages 2145-2154, XP000676670 * the whole document * | 1 | |
| A | WO 91 10140 A (UNIV TECHNOLOGIES INT) | | |
| A | GORDON E M ET AL: "APPLICATIONS OF COMBINATORIAL TECHNOLOGIES TO DRUG DISCOVERY 2. COMBINATORIAL ORGANIC SYNTHESIS, LIBRARY SCREENING STRATEGIES, AND FUTURE DIRECTIONS1" JOURNAL OF MEDICINAL CHEMISTRY, vol. 37, no. 10, 13 May 1994, pages 1385-1401, XP000605162 | | TECHNICAL FIELDS SEARCHED (Int.Cl.6) G06F G01N |

The present search report has been drawn up for all claims

| Place of search | Date of completion of the search | Examiner |
|---|---|---|
| THE HAGUE | 14 April 1998 | Guingale, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 97 40 2620

This annex lists the patent family members relating to the patent documents cited in the above-mentioned European search report.
The members are as contained in the European Patent Office EDP file on
The European Patent Office is in no way liable for these particulars which are merely given for the purpose of information.

14-04-1998

| Patent document cited in search report | Publication date | Patent family member(s) | Publication date |
|---|---|---|---|
| EP 0790567 A | 20-08-97 | WO 9613785 A | 09-05-96 |
| WO 9110140 A | 11-07-91 | AU 7034791 A | 24-07-91 |
| | | CA 2072363 A | 30-06-91 |
| | | EP 0557276 A | 01-09-93 |
| | | HU 65361 A | 02-05-94 |
| | | JP 5503691 T | 17-06-93 |
| | | PT 96440 A | 15-10-91 |
| | | US 5459077 A | 17-10-95 |

For more details about this annex : see Official Journal of the European Patent Office, No. 12/82